(19) Europäisches Patentamt
European Patent Office
Office européen des brevets

(11) EP 0 821 933 A1

# (12) EUROPÄISCHE PATENTANMELDUNG

(43) Veröffentlichungstag:
**04.02.1998 Patentblatt 1998/06**

(51) Int. Cl.[6]: **A61K 7/06**, C08G 69/10, C08G 73/06

(21) Anmeldenummer: **97113351.7**

(22) Anmeldetag: **01.08.1997**

(84) Benannte Vertragsstaaten:
**AT BE CH DE DK ES FI FR GB GR IE IT LI LU MC NL PT SE**

(30) Priorität: **02.08.1996 DE 19631380**

(71) Anmelder:
**BASF AKTIENGESELLSCHAFT**
**67056 Ludwigshafen (DE)**

(72) Erfinder:
- **Nguyen Kim, Son, Dr.**
  **69502 Hemsbach (DE)**
- **Sanner, Axel, Dr.**
  **67227 Frankenthal (DE)**
- **Hössel, Peter, Dr.**
  **67105 Schifferstadt (DE)**
- **Kroner, Matthias, Dr.**
  **67304 Eisenberg (DE)**

(74) Vertreter:
**Kinzebach, Werner, Dr. et al**
**Patentanwälte**
**Reitstötter, Kinzebach und Partner**
**Postfach 86 06 49**
**81633 München (DE)**

## (54) Wasserlösliche oder wasserdispergierbare Polyasparaginsäure-Derivate, ihre Herstellung und ihre Verwendung

(57) Die vorliegende Erfindung betrifft wasserlösliche oder wasserdispergierbare Polyasparaginsäurederivate, ihre Herstellung und ihre Verwendung in der Kosmetik, insbesondere in der Haarkosmetik.

EP 0 821 933 A1

## Beschreibung

Die vorliegende Erfindung betrifft wasserlösliche oder wasserdispergierbare Polyasparaginsäure-Derivate, ihre Herstellung und ihre Verwendung in der Kosmetik.

Synthetisch hergestellte Polyaminosäuren und ihre Derivate sind seit langem bekannt und werden aufgrund ihrer biologischen Verträglichkeit, z.B. für spezielle Anwendungen in der Medizin und Pharmazie verwendet. Abgesehen von Peptiden mit wirkungsspezifischer Sequenz, handelt es sich dabei vor allem um filmbildende Substanzen zur Verbesserung der Handhabbarkeit von pharmazeutischen Zubereitungen, zur Verbesserung ihrer Lagerstabilität und vor allem zur Beeinflussung der Abgabegeschwindigkeit der Wirksubstanzen. Dabei werden z.B. Polymere aus einzelnen Aminosäuren, wie Polyaparaginsäure, Polyglutaminsäure und Polylysin sowie Mischpolymerisate und biologisch gut verträgliche Derivate solcher Polyaminosäuren verwendet.

Die DE-A-37 00 128 beschreibt Poly-(hydroxyalkyl)-aminodicarbonsäurederivate mit biologisch inaktiven Acylgruppen, Verfahren zu ihrer Herstellung und ihre Verwendung-für Wirkstoffdepotzubereitungen mit kontrollierter Wirkstoffabgabe.

Die DE-A-36 12 102 beschreibt lösliche und biologisch abbaubare Mischpolymerisate aus Asparaginsäure- und/oder Glutaminsäure-Einheiten, die reaktive Gruppen, wie z.B. Hydrazid oder Azidgruppen, für die chemische Anbindung von biologisch aktiven Stoffen enthalten.

Die EP-B-0 406 623 beschreibt filmbildende Polyasparaginsäure-Derivate, die durch Umsetzung von Polysuccinimid mit Aminen erhalten werden und Struktureinheiten entsprechend der folgenden Formel

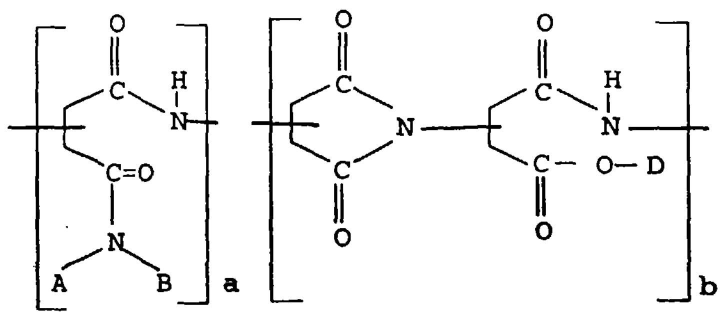

enthalten, in der

A = H oder ein Alkylrest bzw. ein Alkylenrest mit 1 bis 8 C-Atomen ist, der auch verzweigt und noch mit cycloaliphatischen oder aromatichen Resten, wobei der ringförmige Substituent auch Heteroatome enthalten, oder mit R-O-Gruppen, wobei R = H oder gegebenenfalls eine verzweigte Alkylgruppe oder Cycloalkylgruppe mit 1 bis 10 C-Atomen sein kann, substituiert sein kann,

B = H oder ein wie unter A definierter Alkyl- bzw. Alkylenrest ist, der gleich oder verschieden von A sein kann,

D = H oder $NH_4$ oder

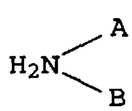

mit den oben angegebenen Bedeutungen von A und B oder ein Alkali und

a = 0,2 bis 1 und

b = 0,8 bis 0

sind, die als Überzugsmittel und/oder Retardierungsmittel für Arzneiformen von therapeutischen Wirkstoffen und für Lebensmittel und Genußmittel verwendet werden. Wie die obige Formel zeigt, sind dabei die Monomereinheiten, die in Salzform vorliegen immer an eine Imidmonomereinheit gebunden. Das stöchiometrische Verhältnis von Monomereinheiten in Salzform zu Imidmonomereinheiten ist auf 1:1 festgelegt.

Auch eine kosmetische Verwendung von Polyaminosäuren und ihren Derivaten ist bereits beschrieben worden. Polypeptide aus Proteinhydrolysaten, z.B. auf Eiweiß- oder Kollagenbasis, sind im Handel erhältlich. So werden z.B. Proteinhydrolysate oder Partialhydrolysate aus Kollagen mit eine Mol-Gew. von 1100 bis 1300 und etwa 8 bis 14 Monomer-Einheiten und deren Natriumsalze (z.B. Nutrilan® der Fa. Grünau) als Schutzkolloide, die nicht schäumen und

nicht waschaktiv sind, aber dispergierende und schmutztragende Eigenschaften aufweisen, z.B. in Kombination mit Tensiden verwendet. Ebenso sind verschiedene Fettsäure-Polypeptid-Kondensationsprodukte als biologisch abbaubare anionische Tenside mit guten Schaum- und Waschvermögen im Handel erhältlich (z.B. Lamepon® der Fa. Grünau).

Die DE-A-22 53 190 beschreibt Polyasparaginsäurederivate mit Säureamid-und Alkali- bzw. Erdalkalicarboxylat-Resten, ihre Herstellung durch Umsetzung von Polysuccinimid mit einem Molekulargewicht von 300 bis 30000 mit einem primären oder sekundären Amin und anschliessender Hydrolyse mit einem Alkali- oder Erdalkalihydroxid oder -carbonat sowie die Verwendung der erhaltenen Produkte als Tenside und Zusatzstoffe für Waschmittel und Kosmetika.

Die JP-A-0624 8072 beschreibt die kosmetische Verwendung von Polyasparaginsäureamiden mit Alkalicarboxylatresten.

Im Bereich der Kosmetik besteht ein grosser Bedarf an in Wasser löslichen oder dispergierbaren Polymeren, die gute biologische Verträglichkeit und biologische Abbaubarkeit sowie konditionierende Eigenschaften aufweisen.

Eine Verwendung der oben beschriebenen Polymere auf Basis von Polyaminosäurederivaten in der Kosmetik als Haarkonditinierungsmittel ist bisher noch nicht beschrieben.

Der vorliegenden Erfindung liegt daher die Aufgabe zugrunde, Polymere auf Basis von Polyaminosäurederivaten zur Verfügung zu stellen, die als Haarkonditionierungsmittel brauchbar sind.

Überraschenderweise wurde nun gefunden, daß diese Aufgabe durch wasserlösliche oder wasserdispergierbare Polyasparaginsäurederivate gelöst wird, welche Umsetzungsprodukte aus Polysuccinimid oder Polyasparaginsäure mit einem Amingemisch, das allgemein mindestens ein kurzkettiges und mindestens ein längerkettiges Amin enthält, sind.

Gegenstand der vorliegenden Erfindung sind daher wasserlösliche und/oder wasserdispergierbare Polyasparaginsäurederivate auf Basis der in der schematisierten Formel I gezeigten Einheiten

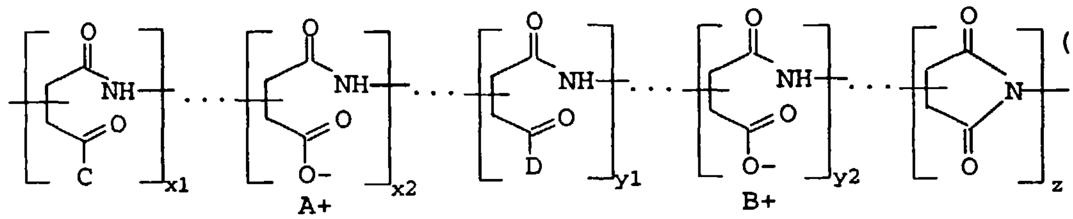 (I)

wobei
die Reihenfolge der Einheiten beliebig ist,
die Summe aus x1 + x2 + y1 + $y^2$ + z = 100 ist und

| | |
|---|---|
| x1 + x2 | für einen Wert von 30 bis 99,9 steht, |
| y1 + y2 | für einen Wert von 0,1 bis 70 steht, |
| z | für einen Wert von 0 bis 20 steht, |
| A | für wenigstens ein primäres, sekundäres oder tertiäres Alkylamin mit 2 bis 6 Kohlenstoffatomen pro Alkylrest steht, wobei die Alkylreste mit 1, 2 oder 3 Gruppen substituiert sein können, die unabhängig voneinander ausgewählt sind unter Hydroxy und Alkoxy, und/oder A für wenigstens ein Diamin der Formel II |

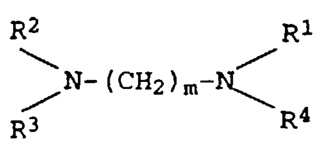 (II)

| | |
|---|---|
| | steht, worin |
| m | für eine ganze Zahl von 2 bis 6 steht, |
| $R^1$ und $R^4$ | gleich oder verschieden sein können und für Wasserstoff oder Alkyl stehen, |
| $R^2$ und $R^3$ | gleich oder verschieden sein können und für Alkyl stehen oder $R^2$ und $R^3$ zusammen mit dem Stickstoffatom, an das sie gebunden sind, für einen 5- bis 7-gliedrigen, gesättigten Heterocyclus der Formel III |

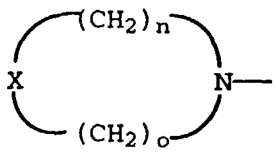

(III)

stehen, worin

| | |
|---|---|
| n und o | gleich oder verschieden sein können und für eine ganze Zahl von 1 bis 5 stehen, |
| X | für $CH_2$, S, O, $NR^5$ oder $NCOR^5$ steht, und |
| $R^5$ | für H oder Alkyl steht, |

und/oder A für wenigstens ein cyclisches Diamin der Formel IIIa steht,

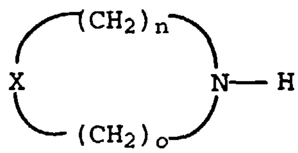

(IIIa)

wobei

| | |
|---|---|
| n und o | die oben angegebene Bedeutung besitzen, |
| X | für $NR^5$ steht, und |
| $R^5$ | für H oder Alkyl steht, |
| B | für wenigstens ein Amin der Formel IV |

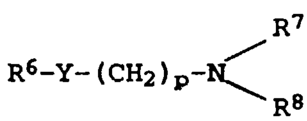

(IV)

steht, wobei der Rest $R^6$-Y-$(CH_2)_p$ 6 bis 24 Kohlenstoffatome aufweist,

| | |
|---|---|
| p | für eine ganze Zahl von 1 bis 23 steht, steht, |
| Y | für $CH_2$, O, NH, CONH , wobei die CO-Gruppe an $R^6$ gebunden ist, oder |

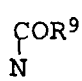

| | |
|---|---|
| | steht, |
| $R^6$ | für Wasserstoff oder den Kohlenwasserstoffrest einer gesättigten oder ungesättigten Fettsäure steht, |
| $R^7$ und $R^8$ | gleich oder verschieden sein können und für Wasserstoff, Alkyl, Hydroxyalkyl oder einen Rest $[CH_2-CH_2-O]_rH$ stehen, worin |
| r | für eine ganze Zahl von 1 bis 30 steht, und |
| $R^9$ | für Alkyl steht, |
| C | für einen von einem Amin des Typs A mit primären oder sekundären Aminogruppen durch Abspaltung eines Aminwasserstoffs abgeleiteten Rest steht, |
| D | für einen von einem Amin des Typs B mit primären oder sekundären Aminogruppen durch Abspaltung eines Aminwasserstoffs abgeleiteten Rest steht, |

wobei mindestens eines der Amine A oder B
eine tertiäre Aminogruppe aufweist;
oder deren Carbonsäure- und Polycarbonsäuresalze oder Quaternierungsprodukte.

Im Rahmen der vorliegenden Erfindung umfasst der Ausdruck "Alkyle" geradkettige und verzweigte Alkylgruppen, vorzugsweise $C_1$-$C_{30}$-Al-kylgruppen. Bevorzugt handelt es sich dabei um geradkettige oder verzweigte $C_1$-$C_8$-Alkyl-, besonders bevorzugt $C_1$-$C_6$-Alkyl- und insbesondere $C_1$-$C_4$-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, n-Hexyl und Ethylhexyl.

Kohlenwasserstoffreste, die sich von einer natürlich vorkommenden, gesättigten oder ungesättigten Fettsäure ableiten, stehen dann beispielswiese für n-Heptyl, n-Octyl, n-Nonyl-, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Docosyl, 9-Hexadecenyl, 9-Octadecenyl oder 13-Docosenyl, 2,4-Hexadienyl und 9,12-Octadienyl.

Geeignete Reste der Formel III sind z.B. Pyrrolidin, Piperidin, Azepan, Imidazolidin, N-Alkylimidazolidin, N-Acylimidazolidin, Oxazolidin, Morpholin, Piperazin, N-Alkylpiperazin, N-Acylpiperazin etc.

Der Rest C leitet sich von einem primären oder sekundären Amin des Typs A ab, bei dem ein Aminwasserstoff in einer Kondensationsreaktion unter Ausbildung einer Arnidbindung abgespalten wurde.

Der Rest D leitet sich von einem primären oder sekundären Amin des Typs B ab, bei dem ein Aminwasserstoff in einer Kondensationsreaktion unter Ausbildung einer Amidbindung abgespalten wurde.

Der Rest $A^+$ leitet sich von einem Amin des Typs A ab, das mit einer Carboxylgruppe ein Ammoniumsalz gebildet hat.

Der Rest $B^+$ leitet sich von einem Amin des Typs B ab, das mit einer Carboxylgruppe ein Ammoniumsalz gebildet hat.

Bei den Endgruppen der Polymere handelt es sich um Resten, die zwei freie terminale Carboxylgruppen oder eine freie terminale Aminogruppe und eine freie Carboxylgruppe aufweisen sowie gegebenenfalls um deren partielle oder vollständige Quaternisierungsprodukte oder gegebenenfalls deren Salze mit Aminen vom Typ A oder B oder gegebenenfalls deren Salze mit zur Neutralisierung verwendeter Carbon- oder Polycarbonsäuren.

Wenn A für ein primäres, sekundäres oder tertiäres Alkylamin mit 2 bis 6 Kohlenstoffatomen steht, handelt es sich dabei vorzugsweise um ein primäres oder tertiäres Amin.

Nach einer bevorzugten Ausführungsform steht A für ein tertiäres Alkylamin, wobei einer der Alkylreste mit einer Hydroxy- oder Alkoxygruppe substituiert ist.

A steht dann beispielsweise für N,N-Dimethylethanolamin, N,N-Dimethylpropanolamin und insbesondere für N,N-Diethylethanolamin.

Vorzugsweise steht A weiterhin für ein Diamin der Formel II mit einer primären und einer tertiären Aminogruppe, wobei $R^1$ und $R^4$ für Wasserstoff stehen, $R^2$ und $R^3$ gleich oder verschieden sein können und für einen kurzkettigen Alkylrest stehen.

A steht dann beispielsweise für N,N-Dimethylethylendiamin, N,N-Diinethyl-1,4-butandiamin, N,N-Dimethyl-1,5-pentandiamin, N,N-Diethylethylendiamin, N,N-Diethylpropylendiamin etc. und insbesondere für N, N-Dimethylpropylendiamin.

Gemäß einer weiteren bevorzugten Ausführungsform steht A für ein Diamin der Formel II mit einer primären und einer tertiären Aminogruppe, worin $R^1$ und $R^4$ für Wasserstoff stehen, $R^2$ und $R^3$ zusammen mit dem Stickstoffatom, an das sie gebunden sind, für einen 5- bis 7-gliedrigen gesättigten Heterocyclus der Formel III stehen, worin X für $CH_2$ oder O steht.

A steht dann beispielsweise für N-(3-Aminoethyl)pyrrolidin, N-(3-Aminopropyl)pyrrolidin oder Morpholinoethylamin.

Gemäß einer weiteren bevorzugten Ausführungsform steht A für einen gesättigten Heterocyclus der Formel IIIa, worin X für $NR^5$ steht und $R^5$ für Wasserstoff oder Alkyl steht. A steht dann beispielsweise für N-Methylpiperazin, N-Ethylpiperazin oder N-Propylpiperazin.

Vorzugsweise ist B ein Amin der Formel IV, worin Y für $CH_2$ steht und $R^7$ und $R^8$ ein Wasserstoffatom bedeuten.

B steht dann beispielsweise für 1-Hexylamin, 1-Heptylamin, 1-Octylamin, 1-Nonylamin, 1-Decylamin, 1-Undecylamin, 1-Undec-10-enylamin, 1-Tridecylamin, 1-Tetradecylamin, 1-Pentadecylamin, 1-Hexadecylamin, 1-Heptadecylamin, 1-Octadecylamin, 1-Octadeca-9,12-dienylamin, 1-Nonadecylamin, 1-Eicosylamin, 1-Eicos-9-enylamin, 1-Heneicosylamin, 1-Docosylamin und insbesondere für 1-Dodecylamin oder für aus natürlich vorkommenden Fettsäuren hergestellte Amingemische. wie z.B. Taigfettamine, die überwiegend gesättigte und ungesättigte $C_{14}$-, $C_{16}$- und $C_{18}$-Alkylamine enthalten oder Kokosamine, die gesättigte, einfach und zweifach ungesättigte $C_6$-$C_{22}$-, vorzugsweise $C_{12}$-$C_{14}$-Alkylamine enthalten. Geeignete Amingemische sind z.B. verschiedene Armeen®-Marken der Fa. AKZO Chemie oder Noram®-Marken der Fa. Ceca.

Gemäß einer weiteren bevorzugten Ausführungsform enthalten die erfindungsgemäßen Polyasparaginsäurederivate als Amin oder Amingemisch vom Typ B tertiäre Fettamine der Formel IV,
worin Y für $CH_2$ steht und

$R^7$ und $R^8$ gleich oder verschieden sein können und für Alkyl, insbesondere kürzerkettige Alkylreste stehen.

B steht dann beispielsweise für N,N-Dimethyl-1-hexylamin, N,N-Diethylhexylamin etc. Insbesondere steht B für dialkylierte Talgfettamine, dialkylierte hydrierte Talgfettamine und dialkylierte Kokosamine und speziell für ein Gemisch aus N,N-Dimethyl$C_{12}$-$C_{14}$-alkylaminen. Geeignete Amine sind z.B. Noram® DM der Fa. Ceca.

Gemäß einer weiteren bevorzugten Ausführungsform enthalten die erfindungsgemäßen Polyasparaginsäurederivate als Amin oder Amingemisch vom Typ B hydroxyethylierte oder ethoxylierte Fettamine der Formel IV, worin Y für $CH_2$ steht und $R^7$ und $R^8$ gleich oder verschieden sein können und für Alkyl oder einen Rest $[CH_2-CH_2-O]_rH$ stehen, worin r für eine ganze Zahl von 1 bis 30 steht. Geeignete Amine sind z.B. Noramox® der Fa. Ceca und insbesondere Noramox O5, ein ethoxyliertes Oleylamin mit 5 Ethylenoxideinheiten.

Gemäß einer weiteren bevorzugten Ausführungsform enthalten die erfindungsgemäßen Polyasparaginsäurederivate als Amin oder Amingemisch vom Typ B ein Fettamin der Formel IV, worin Y für CONH steht.

Gemäß einer weiteren bevorzugten Ausführungsform enthalten die erfindungsgemäßen Polyasparaginsäurederivate als Amin oder Amingemisch vom Typ B ein Diamin der Formel IV, worin Y für NH steht, und $R^7$ und $R^8$ ein Wasserstoffatom bedeuten.

Insbesondere handelt es sich bei $R^6$ dann um einen längerkettigen Alkylrest und B steht dann z.B. für N-Oleyl-1,3-diaminopropan, N-Dodecyl-1,3-diaminopropan oder N-alkylierte 1,3-Diaminopropane mit von Talgfettsäuren oder Kokosfettsäuren abgeleiteten Alkylresten. Geeignete Amine sind z.B. Dinoram®-Marken der Fa. Ceca, Duomeen®-Marken der Fa. Akzo und die Typen 6540, 6560, 6570 und 6572 der Fa. Fina.

Bei den erfindungsgemäßen wasserlöslichen oder wasserdispergierbaren Polyasparaginsäurederivaten handelt es sich insbesondere um Derivate nach der schematisierten Formel 1, wobei

x1 + x2 für einen Wert von 50 bis 97 steht,
y1 + y2 für einen Wert von 3 bis 50 steht,
z für einen Wert von 0 bis 15 steht.

Vorzugsweise beträgt der Molanteil an Ammoniumgruppen in den Polyasparaginsäurederivaten nach der Formel I, ausgedrückt als Summe x2+y2, 50 % und mehr, insbesondere 70 % und mehr. Diese Derivate weisen eine gute biologische Abbaubarkeit auf.

Gemäß einer besonders bevorzugten Ausführungsform handelt es sich bei den erfindungsgemäßen Polyasparaginsäurederivaten um Derivate der Formel I, wobei x1 und y1 gleich 0 sind.

Speziell handelt es sich um Derivate, wobei

A für wenigstens ein tertiäres Amin mit 2 bis 6 Kohlenstoffatomen steht, und/oder für ein Diamin der Formel II

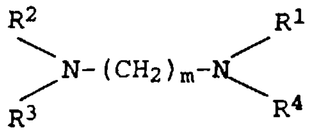

(II)

steht, worin
$R^1$ und $R^4$ gleich oder verschieden sein können und für Alkyl stehen, und
$R^2$, $R^3$, $R^5$, X, m, n und o die zuvor für die bevorzugten Ausführungsformen angegebenen Bedeutungen besitzen,
B für wenigstens ein tertiäres Amin nach Formel IV steht, insbesondere für ein tertiäres Amin, bei dem die Substituenten die für die bevorzugten Ausführungsformen angegebenen Bedeutungen besitzen.

Die Herstellung der erfindungsgemäßen Polyasparaginsäurederivate erfolgt durch Umsetzung von Polyasparaginsäure oder von Polysuccinimid mit mindestens einem Amin vom Typ A und mindestens einem Amin vom Typ B in Wasser als Lösungsmittel. Wird für die Herstellung der erfindungsgemäßen Polyasparaginsäurederivate Polysuccinimid verwendet, so wird dies vorzugsweise durch Polykondensation von Asparaginsäure hergestellt, wobei hochmolekulares Polysuccinimid mit Molekulargewichten von bis zu 100 000 erhalten wird. Die Herstellung erfolgt nach bekannten Verfahren, z.B. durch Polykondensation in Gegenwart von Phosphorsäure, wie z.B. von P. Neri et al in J.Med.Chem.16, 893 (1973) beschrieben.

Zur Herstellung der Polyasparaginsäurederivate wird das Polysuccinimid oder die Polyasparaginsäure, z.B. bei einer Temperatur von 20 bis 70°C, in einem organischen Lösungsmittel, wie N-Methylpyrrolidon, Dimethylformamid,

Butyrolacton oder Tetrahydrofuran, bevorzugt aber in Wasser gegeben. Unter Rühren wird eine Aminmischung mit jeweils einem Amin der vorbeschriebenen Typen A und B zugegeben. Bei einer Temperatur von 30 bis 100°C, vorzugsweise 50 bis 80°C, lässt man, zweckmäßigerweise unter Stickstoffatmosphäre, reagieren, bis praktisch keine Imidstruktur mehr durch IR-Spektroskopie nachweisbar ist. Gegebenenfalls kann die Temperatur auch erhöht und/oder überschüssiges Amin entfernt werden (z.B. durch Destillation). Anschliessend kann das Produkt entweder mit einem Quaternisierungsmittel, wie z.B. Dimethylsulfat, quaternisiert oder mit einer Säure bzw. einer Polycarbonsäure, wie z.B. Milchsäure bzw. Polyasparaginsäure, neutralisiert werden.

Vorzugsweise ist der Anteil von zugesetzten Aminen gleich groß oder grösser als von der Stöchiometrie der Reaktion mit den Monomereinheiten der Polyasparaginsäure oder des Polysuccinimids gefordert wird. Wasserunlösliche Amine können vorzugsweise bereits in neutralisierter Form eingesetzt werden.

Um Polymere mit guten Konditioniereigenschaften zu erhalten, müssen sowohl Amine vom Typ A als auch Amine vom Typ B im Polymer in den vorgenannten Mengen enthalten sein, bevorzugt in Salzform, um gleichzeitig eine gute biologische Abbaubarkeit zu erreichen. Gemäß der vorgenannten, besonders bevorzugten Ausführungsform enthält das Polymer keine Amidgruppen mehr. Die Mengenverhältnisse von Amid-, Salz- und Imideinheiten im Polymer können über die Reaktionsbedingungen gesteuert werden.

Die erfindungsgemäßen wasserdispergierbaren Verbindungen können in Form von wässrigen Mikrodispersionen mit Teilchendurchmessern von üblicherweise 5 bis 100 nm, insbesondere 10 bis 80 nm, und Feststoffgehalten von üblicherweise 0,1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Die Polyasparaginsäurederivate sind aufgrund ihrer ionischen Gruppierungen in der Regel leicht alkohol- und wasserlöslich oder zumindest ohne Zuhilfenahme von Ernulgatoren in Alkohol und Wasser dispergierbar. Geladene Gruppierungen lassen sich aus den vorliegenden tertiären Aminstickstoffatomen entweder durch Protonierung, z.B. mit Alkylierungsmitteln wie $C_1$-$C_4$-Alkylhalogeniden oder -sulfaten erzeugen. Beispiele solcher Alkylierungsmittel sind Ethylchlorid, Ethylbromid, Methylchlorid, Methylbromid, Dimethylsulfat und Diethylsulfat.

Des weiteren können die Polyasparaginsäurederivate auch, wie erwähnt, durch Umsetzung mit einer ein- oder mehrwertigen Carbonsäure, wie z.B. Milchsäure, Zitronensäure, Weinsäure etc., oder durch Umsetzung mit einer Polycarbonsäure, wie z.B. Polyasparaginsäure, Polyglutaminsäure, Carboxymethylcellulose, Polyacrylsäure etc. in leicht wasser- oder alkohollösliche oder dispergierbare Salze überführt werden.

Die erfindungsgemäßen Polyasparaginsäurederivate sind als Hilfsmittel in der Kosmetik und Pharmazie sowie als Beschichtungsmittel für die Textil-, Papier-, Druck-, Agrar- und Klebstoff-Industrie brauchbar. Sie sind insbesondere in der Haarkosmetik als Haarkonditionierungsmittel brauchbar und ziehen leicht auf das Haar auf. Bevorzugt sind Polyasparaginsäurederivate, deren K-Wert (nach Fikentscher, Cellulose Chemie 13 (1932), S. 58-64, 15 bis 90) 25 bis 50 beträgt. Daneben können die Polymere auch in Cremes verwendet werden.

Gegenstand der vorliegenden Erfindung sind auch Haarbehandlungsmittel, die die erfindungsgemäßen Polyasparaginsäurederivate enthalten. Im allgemeinen enthält das Mittel die Polyasparaginsäurederivate in einer Menge von etwa 0,1 bis 30 Gew.-%, bezogen auf das Gesamtgewicht des Mittels.

Die erfindungsgemäßen Haarbehandlungsimittel liegen üblicherweise in Form einer wässrigen Lösung oder Dispersion oder in Form einer wässrig-alkoholischen Lösung vor. Beispiele geeigneter Alkohole sind Ethanol, Propanol, Isopropanol etc. Die Mittel enthalten das Polyasparaginsäurederivat dann in einer Menge von etwa 0,1 bis 25 Gew.-%, bevorzugt 1 bis 15 Gew.-%. Vorzugsweise liegen die Mittel in Form von Shampoos vor.

Weiter können die erfindungsgemäßen Haarbehandlungsmittel allgemein übliche kosmetische Hilfsstoffe, beispielsweise Weichmacher, wie Glycerin und Glycol, Silikone, Emollienzien, Parfüms, UV-Absorber, Farbstoffe, Verdikkungsmittel, antistatische Mittel, Mittel zur Verbesserung der Kämmbarkeit, Konservierungsmittel, Tenside und Schaumstabilisatoren enthalten.

Gewünschtenfalls enthalten die Haarbehandlungsmittel zusätzlich mindestens ein herkömmliches Haarpolymer. Das auf das Gewicht bezogene Mischungsverhältnis von Polyasparaginsäurederivat zu dem anderen Haarpolymer beträgt dann insbesondere 1:0,1 bis 1:2. Das Haarbehandlungsmittel enthält die Polymermischung in einer Menge von 0,1 bis 25 Gew.-%, vorzugsweise 1 bis 15 Gew.-%.

Die erfindungsgemäßen Polyasparaginsäurederivate und diese enthaltende Mittel besitzen den Vorteil, daß sie Haarkonditionierungseigenschaften aufweisen und bioverträglich und biologisch abbaubar sind.

Die Erfindung soll durch die nachfolgenden, nicht einschränkenden Beispiele erläutert werden:

Beispiele

a) Herstellung der Polyasparaginsäurederivate

Herstellungsbeispiel 1:

In einem Dreihaiskolben mit Rührer, Tropftrichter und Rückflußkühler werden 97 g Polysuccinimid und 26,7 g (0,2 mol) $C_{12}$-$C_{14}$-Dimethylamin in 400 g Wasser vorgelegt und unter Stickstoffatmosphäre 16 Stunden bei 60 bis 70°C gerührt. Dann wird der pH-Wert der Lösung auf 9,2 eingestellt und 96,4 g (0,83 mol) Diethylethanolamin werden langsam zugetropft. Anschließend wird das Reaktionsgemisch bei etwa 90°C solange gerührt, bis das IR-Spektrum praktisch keine Imid-Struktur mehr zeigt Bei 110°C wird der Aminüberschuß zusammen mit dem Wasser abdestilliert. Man erhält eine etwa 40%ige Lösung des Polyasparaginsäurederivats.

Analog zu dieser Vorschrift wurden die Beispiele 2 bis 6 der folgenden Tabelle 1 hergestellt.

Tabelle 1

| Beispiel Nr. | Si. mol | $C_{12}/_{14}$-DMA mol | Noram.O5 mol | $C_{12}$-A mol | DEEA mol | DMPDA mol |
|---|---|---|---|---|---|---|
| 1 | 1 | 0,1 | - | - | 0,9 | - |
| 2 | 1 | 0,2 | - | - | 0,83 | - |
| 3 | 1 | - | 0,2 | - | 0,83 | - |
| 4 | 1 | - | 0,2 | - | - | 0,5 |
| 5 | 1 | - | - | 0,1 | - | 0,42 |
| 6 | 1 | - | - | 0,3 | 0,75 | - |

Si.: Succinimideinheiten im Polymer
$C_{12}/_{14}$-DMA: $C_{12}$-$C_{14}$-Dimethylamin
Noram.O5: Noramox® O5 (ethoxyliertes Oleylamin mit 5 EO-Einheiten der Fa. CECA)
$C_{12}$-A: Dodecylamin
DEEA: Diethylethanolamin
DMPDA: N,N-Dimethylpropylendiamin

b) Biologische Abbaubarkeit

Die biologische Abbaubarkeit wurde nach den OECD-Prüfvorschriften OECD 302 B (Zahn-Wellens-Test) und OECD 301 B (Sturm-Test) ermittelt. Ersterer bestimmt den Grad des oxidativen Abbaus durch die Menge an freigesetztem $CO_2$ in einem bestimmten Zeitraum (60 Tage), letzterer die Eliminierbarkeit des Stoffes selbst. Die Ergebnisse der Untersuchung sind in Tabelle 2 wiedergegeben.

Tabelle 2

| Bsp-Nr. nach Tabelle 1 | biologische Abbaubarkeit | |
|---|---|---|
| | $CO_2$-Menge [%] | Eliminierbarkeit [%] |
| 1 | 92% (60d) | >93% |
| 2 | 87% (60d) | >90% |
| 3 | 90% (60d) | >95% |
| 4 | 82% (60d) | >85% |
| 5 | 85% (60d) | >90% |
| 6 | 87% (60d) | >90% |

c) Anwendungsbeispiele

Um die Verwendung der erfindungsgemäßen Polyasparaginsäurederivate als Haarkonditionerpolymer zu demonstrieren, wurden die folgenden Polymerlösungen und Polymer/Tensid-Lösungen hergestellt und anschließend die Naßkämmbarkeit von mit ihnen behandelten Haarsträhnen bestimmt:

| A) Polymerlösung: | |
|---|---|
| Polymer 1-6 nach Tabelle 1 | 1 Gew.-% |
| Konservierungsmittel auf Benzylalkohol/Isothiazolon-Basis | 0,1 Gew.-% |
| Wasser | 98,9 Gew.-% |

| B) Polymer/Tensid-Lösung: | |
|---|---|
| Polymer 1-6 nach Tabelle 1 | 1 Gew.-% |
| Texapon® NSO (ethoxyliertes Nonylphenolsulfonat der Fa. Henkel KgaA) | 10 Gew.-% |
| Konservierungsmittel auf Benzylalkohol/Isothiazolon-Basis | 0,1 Gew.-% |
| Wasser | 88,9 Gew.-% |

Naßkämmbarkeit nach Auswaschen der Polymerlösung mit handwarmen Trinkwasser:
Je eine Haarsträhne wird 30 Minuten lang vollständig in folgende Lösungen eingetaucht:

100 g vollentsaiztes Wasser (= Blindwert für Polymerlösungen A)
100 g 1%ige Luviquat® FC 370 (Copolymerisat aus Vinylpyrrolidon und Vinylimidazol quarterniert mit Dimethylsulfat der Fa. BASF AG)
100 g zu untersuchende Polymerlösungen A)

Naßkämmbarkeit nach Auswaschen der Polymer/Tensid-Lösung mit handwarmen Trinkwasser:
Je eine Haarsträhne wird 30 Minuten lang vollständig in folgende Lösungen eingetaucht:

100 g 10%ige Texapon® NSO (= Blindwert für Polymerlösungen B)
100 g 1% Luviquat® FC 370 in einer Tensidlösung (10%ige Texapon® NSO)
100 g zu untersuchende Polymer/Tensid-Lösungen B)

Nach leichtem Abstreifen der Haare zwischen den Fingern werden die Strähnen jeweils eine Minute lang unter handwarmen Trinkwasser (30-40°C) ausgespült. Mit einem feinzinkigen Kamm der Fa. Hercules (Zinkenabstand 1 mm) wird die Strähne im Klimaraum gekämmt und beurteilt: Alle Polymerlösungen A) und Polymer/Tensidlösungen B) der Beispiele 1 bis 6 nach Tabelle 1 zeigen mindestens genau so gute Konditionierungseigenschaften wie Luviquat® FC 370.

**Patentansprüche**

1. Wasserlösliche und/oder wasserdispergierbare Polyasparaginsäurederivate auf Basis der in der schematisierten Formel I gezeigten Einheiten

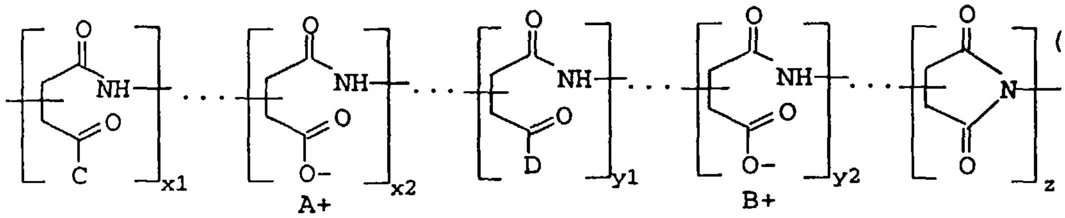

(I)

wobei
die Reihenfolge der Einheiten beliebig ist,
die Summe aus x1 + x2 + y1 + y2 + z = 100 ist und

x1 + x2 für einen Wert von 30 bis 99,9 steht,
y1 + y2 für einen Wert von 0,1 bis 70 steht,
z für einen Wert von 0 bis 20 steht,
A für wenigstens ein primäres, sekundäres oder tertiäres Alkylamin mit 2 bis 6 Kohlenstoffatomen pro Alkylrest steht, wobei die Alkylreste mit 1, 2 oder 3 Gruppen substituiert sein können, die unabhängig voneinander ausgewählt sind unter Hydroxy und Alkoxy, und/oder A für wenigstens ein Diamin der Formel II

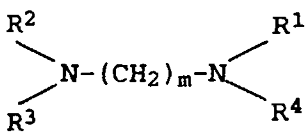

(II)

steht, worin
m für eine ganze Zahl von 2 bis 6 steht,
$R^1$ und $R^4$ gleich oder verschieden sein können und für Wasserstoff oder Alkyl stehen,
$R^2$ und $R^3$ gleich oder verschieden sein können und für Alkyl stehen oder $R^2$ und $R^3$ zusammen mit dem Stickstoffatom, an das sie gebunden sind, für einen 5- bis 7-gliedrigen, gesättigten Heterocyclus der Formel III

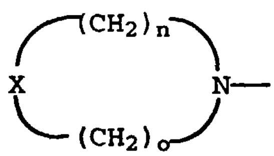

(III)

stehen, worin
n und o gleich oder verschieden sein können und für eine ganze Zahl von 1 bis 5 stehen,
X für $CH_2$, S, O, $NR^5$ oder $NCOR^5$ steht, und
$R^5$ für H oder Alkyl steht,

und/oder A für wenigstens ein cyclisches Diamin der Formel IIIa steht,

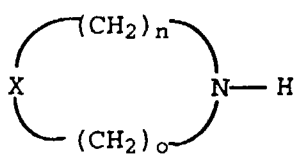

(IIIa)

wobei

| | |
|---|---|
| n und o | die oben angegebene Bedeutung besitzen, |
| X | für $NR^5$ steht, und |
| $R^5$ | für H oder Alkyl steht, |
| B | für wenigstens ein Amin der Formel IV |

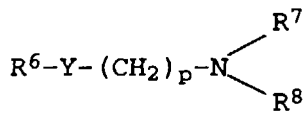

(IV)

| | |
|---|---|
| | steht, wobei der Rest $R^6$-Y-$(CH_2)_p$ 6 bis 24 Kohlenstoffatome aufweist, |
| p | für eine ganze Zahl von 1 bis 23 steht, |
| Y | für $CH_2$, O, NH, CONH , wobei die CO-Gruppe an $R^6$ gebunden ist, oder |

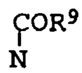

| | |
|---|---|
| | steht, |
| $R^6$ | für Wasserstoff oder den Kohlenwasserstoffrest einer gesättigten oder ungesättigten Fettsäure steht, |
| $R^7$ und $R^8$ | gleich oder verschieden sein können und für Wasserstoff, Alkyl, Hydroxyalkyl oder einen Rest $\{CH_2-CH_2-O\}_rH$ stehen, worin |
| r | für eine ganze Zahl von 1 bis 30 steht, und |
| $R^9$ | für Alkyl steht, |
| C | für einen von einem Amin des Typs A mit primären oder sekundären Aminogruppen durch Abspaltung eines Aminwasserstoffs abgeleiteten Rest steht, |
| D | für einen von einem Amin des Typs B mit primären oder sekundären Aminogruppen durch Abspaltung eines Aminwasserstoffs abgeleiteten Rest steht, |

wobei mindestens eines der Amine A oder B
eine tertiäre Aminogruppe aufweist;
oder deren Carbonsäure- und Polycarbonsäuresalze oder Quaternisierungsprodukte.

**2.** Polyasparaginsäurederivate nach Anspruch 1, wobei

| | |
|---|---|
| x1 + x2 | für einen Wert von 50 bis 97 steht, |
| y1 + y2 | für einen Wert von 3 bis 50 steht, |
| z | für einen Wert von 0 bis 15 steht. |

**3.** Polyasparaginsäurederivate nach einem der Ansprüche 1 oder 2, wobei die Summe x2 + y2 für einen Wert von 50 oder grösser, bevorzugt für einen Wert von 70 oder grösser, steht.

**4.** Polyasparaginsäurederivate nach einem der Ansprüche 1 bis 3, wobei in der Formel I x1 und y1 = O sind.

**5.** Polyasparaginsäurederivate nach einem der Ansprüche 1 bis 4, wobei

| | |
|---|---|
| A | für wenigstens ein tertiäres Alkylamin mit 2 bis 6 Kohlenstoffatomen steht, wobei die Alkylreste mit 1, 2 oder 3 Gruppen substituiert sein können, die unabhängig voneinander ausgewählt sind unter Hydroxy und Alkoxy, und/oder A für ein Diamin der Formel II steht, worin |
| $R^1$ und $R^4$ | gleich oder verschieden sein können und für Alkyl stehen, und |
| $R^2$, $R^3$, $R^5$, X, m, n und o | die in Anspruch 1 angegebenen Bedeutungen besitzen, und |
| B | für wenigstens ein tertiäres Amin der Formel IV steht. |

**6.** Polyasparaginsäurederivate nach einem der Ansprüche 1 bis 5, deren K-Wert 15 bis 90, bevorzugt 25 bis 50 beträgt.

7. Verfahren zur Herstellung der Polyasparaginsäurederivate nach einem der Ansprüche 1 bis 6, dadurch gekennzeichnet, daß man Polyasparaginsäure oder Polysuccinimid mit mindestens einem Amin vom Typ A und mindestens einem Amin vom Typ B zur Reaktion bringt.

8. Verfahren nach Anspruch 7, dadurch gekennzeichnet, daß man die Amine vom Typ A und Typ B in zumindest stöchiometrischer Menge verwendet.

9. Verfahren nach einem der Ansprüche 7 oder 8, dadurch gekennzeichnet, daß das erhaltene Polyasparaginsäurederivat mit einem Quaternisierungsmittel umgesetzt wird.

10. Verfahren nach einem der Ansprüche 7 oder 8, dadurch gekennzeichnet, daß das erhaltene Polyasparaginsäurederivat mit einer Carbonsäure oder Polycarbonsäure umgesetzt wird.

11. Verwendung der Polyasparaginsäurederivate nach einem der Ansprüche 1 bis 6 als Hilfsmittel in der Kosmetik, insbesondere in der Haarkosmetik.

12. Verwendung nach Anspruch 11 als Haarconditioner.

13. Haarbehandlungsmittel, das wenigstens ein Polyasparaginsäurederivat nach einem der Ansprüche 1 bis 6 enthält.

14. Haarbehandlungsmittel nach Anspruch 13, das das/die Polyasparaginsäurederivat(e) in einer Menge von 0,1 bis 25 Gew.-%, vorzugsweise 1 bis 15 Gew.-%, enthält.

15. Haarbehandlungsmittel nach einem der Ansprüche 13 oder 14, das weitere übliche kosmetische Hilfsstoffe, ausgewählt unter Weichmachern, Silikonen, Emollienzien, Parfüms, UV-Absorbern, Farbstoffen, Verdickungsmitteln, Antistatika, Mitteln zur Verbesserung der Kämmbarkeit, Konservierungsmitteln und Schaumstabilisatoren enthält.

16. Haarbehandlungsmittel nach einem der Ansprüche 13 bis 15, das zusätzlich mindestens ein anderes Haarpolymer enthält.

17. Haarbehandlungsmittel nach einem der Ansprüche 13 bis 16, das in Form einer wässrigen Lösung oder Dispersion oder einer wässrig-alkoholischen Lösung vorliegt.

18. Haarbehandlungsmittel nach Anspruch 17 in Form eines Shampoos.

Europäisches Patentamt

# EUROPÄISCHER RECHERCHENBERICHT

Nummer der Anmeldung
EP 97 11 3351

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,X | EP 0 767 191 A (MITSUI TOATSU CHEMICALS)<br>* das ganze Dokument *<br>--- | 1-18 | A61K7/06<br>C08G69/10<br>C08G73/06 |
| D,A | DE 36 12 102 A (CESKOSLOVENSKA AKADEMIE VED)<br>* Ansprüche *<br>--- | 1,7 | |
| D,A | EP 0 406 623 A (ROEHM GMBH)<br>* Seite 4, Zeile 20 - Seite 5, Zeile 33 *<br>--- | 1,7 | |
| D,A | DATABASE WPI<br>Section Ch, Week 9440<br>Derwent Publications Ltd., London, GB;<br>Class A23, AN 94-322255<br>XP002045386<br>& JP 06 248 072 A (AJINOMOTO KK) ,<br>6.September 1994<br>* Zusammenfassung *<br>--- | 1,7,13 | |
| A | US 3 846 380 A (Y. FUJIMOTO ET AL.)<br>* Spalte 3, Zeile 65 - Spalte 4, Zeile 37 *<br>--- | 1,7 | RECHERCHIERTE SACHGEBIETE (Int.Cl.6) |
| A | GB 2 005 705 A (L'OREAL)<br>* Ansprüche *<br>--- | 1-18 | A61K<br>C08G |
| A | FR 2 424 292 A (L'OREAL)<br>* Ansprüche *<br>--- | 1-18 | |
| A | US 4 735 797 A (J. GROLLIER ET AL.)<br>--- | 1-18 | |
| A | E. W. NEUSE ET AL.: "Water-soluble polyamides as potential drug carriers"<br>DIE ANGEWANDTE MAKROMOLEKULARE CHEMIE,<br>Bd. 192, Nr. 3300, 1991, BASEL,<br>Seiten 35-50, XP002045582<br>* das ganze Dokument *<br>----- | 1-18 | |

Der vorliegende Recherchenbericht wurde für alle Patentansprüche erstellt

| Recherchenort | Abschlußdatum der Recherche | Prüfer |
|---|---|---|
| BERLIN | 6.November 1997 | Boeker, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)